# EUROPEAN PATENT SPECIFICATION

(11) **EP 0 725 149 B1**
(45) Date of publication and mention of the grant of the patent: **09.01.2002**
(21) Application number: 95907490.7
(22) Date of filing: 10.08.1993
(51) Int. Cl.: C12Q 1/68, C12Q 1/28, C12Q 1/42

(54) **METHOD OF DETECTING NUCLEIC ACID**
VERFAHREN ZUR BESTIMMUNG VON NUKLEINSÄUREN
PROCEDE DE DETECTION D'UN ACIDE NUCLEIQUE

(43) Date of publication of application: 07.08.1996
(73) Proprietor: FUSO PHARMACEUTICAL INDUSTRIES LTD., Osaka-shi, Osaka 541 (JP)
(72) Inventor: MATSUHISA, Akio, Higashinari-ku, Osaka-shi, Osaja 537 (JP); SHIBA, Kiyotaka, Tokyo 170 (JP); MIKAWA, Yoshikazu, San Diego, CA 90122 (US); KISHI, Yuichiro, Wakayama 640 (JP)
(74) Representative: VOSSIUS & PARTNER
(86) International application number: JP9301124
(87) International publication number: WO9504832

(56) References cited:
- WO-A-88/08036
- JP-A- 1 124 400
- JP-A- 54 143 297
- US-A- 5 092 992
- BRAUNLIN W H ET AL: "EQUILIBRIUM DIALYSUS STUDIES OF POLYAMINE BINDING TO DNA" BIOPOLYMERS, vol. 21, no. 7, 1 July 1982, pages 1301-1314, XP000572564
- CHEMICAL ABSTRACTS, vol. 115, no. 11, 16 September 1991 Columbus, Ohio, US; abstract no. 107800, KAWAKATSU, KUNIO ET AL: "Reagents and method for detecting nucleic acids by hybridization assay" XP002107110 & JP 03 128000 A (SANYO CHEMICAL INDUSTRIES, LTD., JAPAN)
- SCHMID N & BEHR J-P: "Location of spermine and other polyamines on DNA as revealed by photoaffinity cleavage with polyaminobenzenediazonium salts" BIOCHEMISTRY, vol. 30, - 1991 pages 4357-4361, XP002107081

## Description

### FIELD OF THE INVENTION

The present invention relates to a method of detecting a nucleic acid using property in which a polyamine binds electrostatically to a nucleic acid.

### DESCRIPTION OF THE PRIOR ART

As a conventional method of detection of nucleic acids, methods using ethidium bromide have been widely adopted. The known method comprises intercalation of ethidium bromide into a nucleic acid so as to emit fluorescence by which a location of a nucleic acid is detected. However, according to the method it is very inconvenient that fluorescence cannot be detected in the light and the locations of nucleic acids can be detected only by comparing the original gel to a photograph thereof which was taken under ultraviolet radiation in the dark. And also, ethidium bromide requires careful handling because of its strong carcinogenic property (cf. Gene Operation Manual, page 2, lines 18 - 21, Kodansha Scientific Press). Alternatively, methods using anionic gold colloid and cationic iron colloid cacodylate are also known (Gene Anal. Techn. pages 1 to 5, 1986), however, the sensitivity of the methods is low. In addition, since the method using gold colloid is a kind of back ground staining system, detection of a nucleic acid is not easy and also hybridization after the staining is disadvantageously affected. In the method using iron colloid, there is a disadvantage that staining after hybridization cannot be carried out. The development of a operably easy, safe, simple and reproducible method for detecting a nucleic acid has been expected for a long time.
It is already known that a polyamine can bind to DNA and also has affinity for RNA (cf. Biochemical Journal, 123, 811 - 815 (1967), Journal of Molecular Biology, 24, 113 - 122 (1967), ibid., 42, 363 - 373 (1969) and ibid., 121, 327 - 337 (1987)). And also, in order to detect target polynucleotides, it is known to use probes which bond covalently to polynucleotide complementary to protein modified with polyamine (Japanese Patent Publication (in Japanese) A: TOKUKOHYOSHO 60-501488 (1985), to which corresponding, Japanese Patent Publication B: TOKUKOHEI 2-59720 (1990) and its divisional application, Japanese Patent Publication A: TOKUKAIHEI 1-124400 (1989)). However, any method of detecting a nucleic acid is not known which comprises forming a complex of a labeled polyamine and a nucleic acid.

### SUMMARY OF THE INVENTION

The object of the present invention is thus to provide
(1) a method of detecting a nucleic acid which comprises bringing a solid carrier suspected to carry or contain a nucleic acid into contact with a polyamine to which a label capable of generating a detectable signal or a precursor thereof is bound, to form an electrostatically bonded complex between said nucleic acid and said polyamine, said precursor converting into said label, if used, removing the polyamine which has not formed any electrostatically bonded complex before or after the conversion of said precursor and then detecting said label, and
(2) a method of differentiating a target nucleic acid from any nucleic acid other than said target nucleic acid which comprises combining the following steps (i) and (ii) in voluntary order:
   (i) The first step which comprises bringing a solid carrier suspected to carry or contain a target nucleic acid, into contact with a probe to which a label capable of generating a detectable signal or a precursor thereof is bound, being capable of hybridizing with said target nucleic acid under hybridization conditions, to form a hybrid, said precursor converting into said label, if used, removing said probe which has not formed any hybrid before or after the conversion of said precursor and then detecting said label, and
   (ii) the second step which comprises bringing a solid carrier suspected to carry or contain a target nucleic acid, into contact with a polyamine to which a label capable of generating a detectable signal or a precursor thereof is bound, to form an electrostatically bonded complex between said nucleic acid and said polyamine, said precursor converting into said label, if used, removing said polyamine which has not formed any electrostatically bonded complex before or after the conversion of said precursor and then detecting said label, and
(3) A kit for detecting a nucleic acid comprising
   (i) a polyamine-labeled enzyme and
   (ii) a chromogen which generates a label by enzymatic action.

The terms used herein are defined as follows:

The term "a nucleic acid" means a polymer in which nucleotides consisting of a purine or pyrimidine base, a pentose and phosphoric acid as a basic unit are polymerized by ester linkages of the phosphoric acids. As a base, there are included adenine, cytosine, guanine, thymine and uracil, and modified bases thereof. Nucleic acids are distinguished by a sugar moiety into DNA and RNA, by the number of strands into single and double stranded nucleic acids and also by steric structure.

As a "solid carrier", there may be exemplified a thin plate, a sheet, a strip, a slab, film, membrane, etc. There are typically mentioned agarose gel, nylon membrane, filter paper, nitrocellulose membrane, etc.

The term "a label" means a substance which generates a measurable signal and includes an enzyme (in corporation with a substrate), a spin compound, a radioactive nuclear atom, a fluorescent substance, a chemiluminescent substance, an absorption substance, etc, with an enzyme being preferred. As the enzyme there may be included peroxidase, β-galactosidase, glucose oxidase, alkaline phosphatase, glucose-6-phosphate dehydrogenase, etc. As a method for determination of enzyme activity, there may be mentioned absorption method, fluorescence method and chemiluminescence method. For example, in the absorption method, peroxidase is used in corporation with a chromogen, such as 2,2'-azinodi(3-ethylbenzthiazolin)-6'-sulfonate (ABTS) and the color developed is measured by the absorption method. Glucose oxidase produces hydrogen peroxide from glucose as a substrate and therefore, it can be determined by the same way as above by conjugating with peroxidase. β-galactosidase can be determined using o-nitrophenyl-β-D-galactoside as a substrate. In the fluorescence method, for example, peroxidase can be determined using p-hydroxyphenyl-propionic acid as a substrate, β-galactosidase can be determined using fluorescein-β-galactosidase or 4-methylunbelliferyl-β-D-galactoside as a substrate and phosphatase can be measured using unbelliferyl phosphate or bromochloroindolyl phosphate/nitroblue-tetrazolium as a substrate. In the chemiluminescence method, for example, peroxidase can be measured using luminol and hydrogen peroxide as substrates. A label (e.g. an enzyme) may be bound to a polyamine by a crosslinking agent such as benzoquinone (quinhydrone), bis[2-(succinimidocarbonyloxy)ethyl]sulfon (BSOCOES), bis(sulfosuccinimidyl)-suberate (BS3), 1,2-difluoro-2,4-dinitrobenzene (DFDNB), 4,4'-diisothiocyano-2,2'-disulfostilbene disodium salt (CDIDS), dimethyl adipin-imidate dihydrochloride, N-(γ-maleimidobutyryloxy)sucoinimide (GMBS), N-(4-azidophenylthio)phthalimide (APTP), N-succinimidyl-6-(4'-azido-phenyl)-1,3'-dithiopropionate (SADP), pyridine disulfide, thiophthalimide, etc.

The term "a precursor of a label" means, for example, a label blocked with a blocking substance, which can be converted into the detectable label .

The term "signal" means visible ray, fluorescence, radioactive ray, or other electromagnetic waves and the like. A measured value of signal is to be related to an amount of a substance which emits the signal.

The term "detecting a label" is to detect a signal by physical or technical means.

The term "polyamine" herein means a compound which has an aliphatic bone having 2 or more primary amino groups and includes naturally occurring (a living body) amines and synthetic polymers. In general, primary amino groups are at both ends of a chain of 3 to 50 carbon atoms, preferably, about 6 to about 15 or about 20 carbon atoms, and the chain may be interrupted by one or more imino groups. As a typical naturally occurring polyamine, there may be included 1,3-diaminopropane, putrescine, cadaverine, norspermidine, spermidine, homospermidine, aminopropylcadaverine, telmine, spermine, thermospermine, canavalmine, aminopentylnorspermidine, N,N'-bis(aminopropyl)cadaverine, caldopentamine, homocaldopentamine, caldohexamine, etc. A typical synthetic polyamine includes diethylenetriamine, triethylenetetramine, tetraethylenepentamine, pentaethylenehexamine, hexamethylenediamine, polyethyleneimine (an average molcular weight is about 10,000 - about 200,000, preferably, about 20,000 - about 100,000, for example, about 50,000 - about 60,000, e.g. Polymin G 35 by BASF AG), etc.

The word "complex" (a verb) or "complex" (a noun) means to bond electrostatically and/or physically or a electrostatically and/or physically bonded product and does not mean to bond covalently or a covalently bonded product. "Complex" is generally reversible, and can be easily dissociated.

The term "hybridize" or "hybridization" means that, when two single-stranded polynucleotides are complementary or almost complementary (for example, more than 70 %, preferably, more than 80 or 85 %, especially, more than 90 or 95 %), they bind to form a double-stranded polynucleotide.

"Under hybridization conditions" means the conditions under which polynucleotides capable of hybridizing can produce a hybrid. In general, the conditions mean that a temperature is below about 70 °C, preferably below about 60 °C, generally below about 40 to 55 °C and a period that is short enough for hybridization.

"Target" presents a subject to be interested.

"Probe" is a DNA which is highly complementary to the target DNA and a part thereof and, in general, is shorter than the target DNA and has about 5 - about 50 bases, preferably about 10 - about 40 bases, for example, about 20 - about 30 bases.

### BRIEF DESCRIPTION OF THE DRAWINGS

Figure 1 shows the result of detection of λDNA in Example 1.

Figure 2 shows the result of detection of Escherichia coli rRNA in Example 1.

Figure 3 represents a photograph of electrophoresis of λHind III DNA in Example 1.

Figure 4 represents a photograph of electrophoresis showing the result (the color development pattern according to the invention) of splitting a DNA fragment inserted into pBR322 with the restriction enzyme .

Figure 5 represents a photograph of electrophoresis showing the result (the X-ray profile) of splitting a DNA fragment inserted into pBR322 with the restriction enzyme.

Figure 6 represents a photograph of electrophoresis of λHind III DNA in Example 1.

Figure 7 like figure 6 in Example 1 represents the photograph of electrophoresis of λHind III DNA in Example 1.

Figure 8 shows the difference in color in Figure 7.

### DETAILED DESCRIPTION OF PREFERRED EMBODIMENTS

A typical method of detecting a nucleic acid according to the invention may be summarized as follows:

In an example of a complex consisting of a polyamine and an enzyme using benzoquinone, the reaction is considered to proceed according to the following scheme. 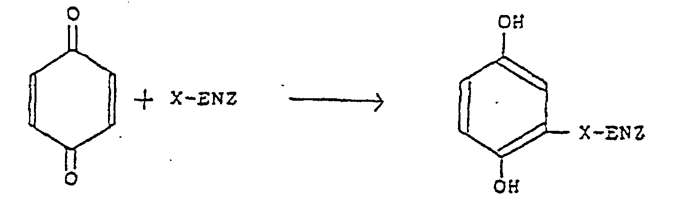 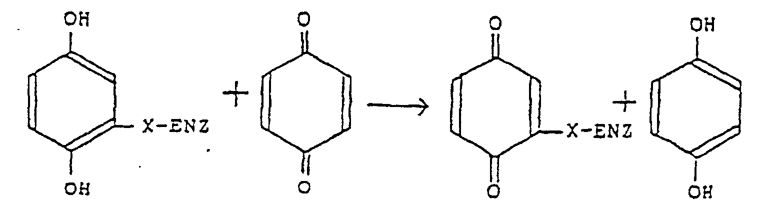 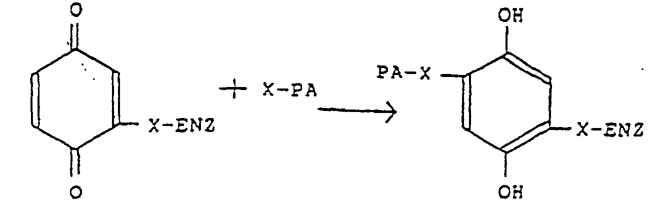 in which ENZ represents a residue that is deleted by an amino group from an enzyme; PA represents a residue that is deleted by an amino group from a polyamine; X represents a primary amine or a secondary amine.

The reaction is, for example, carried out as follows: To benzoquinone (about 1 part) is added an enzyme (about 150 parts) which has been previously purified, for example, by such a mean as dialysis, and preferably at a somewhat elevated temperature to give a reaction mixture of wine-red color, which was subjected to such a method as gel permeation chromatography and fractionated into a purple, a yellow and a wine-red fraction. The wine-red fraction was reacted with a polyamine (one of several parts of the enzyme) in the presence of a weak base while heating slightly to give an enzyme-labeled polyamine after suitable purification.

Detection of a nucleic acid is for example performed as follows:
(a) A nucleic acid specimen is dot-spotted on a membrane or is electrotransferred to a membrane after subjecting to gel electrophoresis and then the membrane is fixed by baking. After treating the membrane with bovine serum albumin in a buffer, a labeled polyamine or a label-precursor labeled polyamine is reached therewith. After washing, the label is detected and, if a label-precursor is used, the label-precursor is converted into the label, and the label is detected.
(b) DNA fragments are subjected to an agarose gel electrophoresis and then Southern blotting. After prehybridization with radiolabeled probes while heating, the DNA fragments are hybridized. After washing, the position of DNA is detected using an X-ray film. Then the DNA is reacted with a labeled or a precursor-labeled polyamine and then a product thereof is developed with a suitable system to detect.
(c) DNA fragments are fixed on a membrane by dot blotting or by Southern blotting after subjecting to agarose gel electrophoresis and after prehybridization, they are hybridized with Bio-DNA probes. After washing and blocking, the resulting product is reacted with an enzyme-labeled streptavidin and after washing and preincubation, in addition, another enzyme-labeled polyamine is reacted therewith. After washing, the resultant hybrid'is subjected to color developments for each enzyme to give two different color patterns corresponding to hybridized DNA and non-hybridized DNA. For example, alkaline phosphatase/BCIP-NBT and peroxidase/DAB systems are developed into purple and brown, respectively. In such a method, the order of treatments with a probe and a polyamine may be reversible.

Such a membrane as treated above develops color in itself, but not as an X-ray film and therefore, a subject nucleic acid can be directly detected on the membrane. In addition, in the PCR method, amplification products having similar molecular weights, but having different sequences, can be distinguished from each other by hybridization.

The method of detecting a nucleic acid according to the invention has the following advantages:

The method requires no dangerous reagents and can result in high sensitivity (i.e. picogram order in DNA and 10 picogram order in RNA). And also operation of the method is very easy, and if the reagents to be used in the method are incorporated into a kit, it becomes easier.

### Examples

The present invention is illustrated by the following specific embodiments.

### Example 1

### Production of peroxidase- or alkaline phosphatase-labeled polyethyleneimine

Alkaline phosphatase (CIP, Boehringer-Mannheim GmbH, Grade 1), 0.81 ml/4.05 mg/7500 unit or horseradish peroxidase (Boehringer-Mannheim GmbH) is dialyzed against 0.1 M phosphate buffer at 40 °C overnight and then, according to a method as described in Immuno Chemistry, Vol. 14, 767-774 (1977), reacted with p-benzoquinone (120 µl/30 mg/ethanol) at 37 °C for 60 minutes in the dark. After subjecting to gel permeation chromatography on Sephadex G-25, wine-red fractions (2.7 ml) were taken up and 300 µl of 1M NaHCO₃ (pH 9.0), 30 µl of polyethyleneimine (Epomin) were added thereto and fully mixed. The resultant mixture was reacted at 37 °C in the dark overnight, and then dialysed against 5 mM phosphate buffer (pH 6.8) to give an enzyme-polyethyleneimine conjugate, which was stable at 4 °C more than 1 year.

### Nucleic acid blotting on a membrane

Denatured λHind III DNA or Escherichia coli K-12 rRNA was dotblotted on a nylon membrane (Biodyne A, Pall Co. Ltd.) as described in "Molecular Cloning", 1982, Cold Spring Harbour Laboratory. A clone of Staphylococcus aureus genome DNA which was random-cloned into a vector pBR322 was amplified according to a method known to those skilled in the art, extracted and then split with Hind III. Specimens were fractionated by 1 % agarose gel electrophoresis and the isolates were electroblotted on a membrane (40V, for 4 hours). λHind III DNA was also blotted as above. The DNA or RNA specimen was subjected to baking (80 °C, 2 hours) to fix on the membrane.

### Detection of nucleic acid blotted on membrane

A membrane containing fixed DNA or RNA was soaked in 5mM phosphate buffer-1% bovine serum albumin (BSA, Sigma, Fraction V), incubated at room temperature for 60 minutes and then alkaline phosphatase (or peroxidase)-labelled polyethyleneimine was added in a ratio of 100 µl/20 ml thereto and incubated at 37 °C for 120 minutes. The membrane was washed 3 times with 5mM phosphate buffer-1 % BSA-1 % Tween 20 for 10 minutes and rinsed with 0.1 M Tris HCl (pH 9.5)-10 mM MgCl₂.
The membrane was developed with 50 ml of a staining solution consisting of 0.1M Tris HCl (pH 9.5)-10 mM MgCl₂ containing each 300 µl of bromochloroindolyl phosphate (BCIP: 75 mg/ml) and nitro blue tetrazolyium (NBT: 50 mg/ml).

### Southern Blotting Hybridization

Nick translation reaction was performed using a Staphylococcus aureus genome kbp fragment DNA according to a conventional technique with ³²P-dCTP or Bio-11-dUTP (BRL) to prepare ³²P-DNA or Bio-DNA. The membrane was treated for hybridization with hybridization buffer (5 x SSC, 0.1 % BSA, 0.1 % Tween 20) containing denatured probes at 42 - 55 °C overnight. Then the membrane was washed with 0.16 x SSC-0.1 % Tween 20 at 55 °C for 30 minutes (The endpoint of washing is referred to as "the point A").

When ³²P-DNA probe was used, at the point A, the moist membrane was wrapped in polyethylene film and the signal was detected by exposing to an X-ray film. Then, the membrane was treated for blocking with 3 % BSA - 0.1 % Tween 20 - phosphate buffer saline (PBS) at 42 °C for more than 3 hours, followed by adding 0.1 % BSA - 0.1 % Tween - PBS containing 100 µl of peroxidase-labelled polyethyleneimine per sheet, the membrane was reacted for 2 - 3 hours. The membrane was washed 3 times for 20 minutes with 0.1 % Tween 20 - PBS and developed with a diaminobenzidine (DAB) - H₂O₂ system.

When Bio - DNA probe was used, after the point A, the membrane was treated with PBS - 0.5 % Tween 20 at room temperature for 60 minutes. After treatment with PBS - 0.05 % Tween 20 containing alkaline phosphatase-labeled strept-avidine at room temperature for 20 minutes, the membrane was washed 2 times with PBS - 0.5 % Tween 20 for 10. minutes. After preincubation with PBS - 0.1 % BSA - 0.1 % Tween 20 at room temperature for 20 minutes, and addition of peroxidase-labeled polyethyleneimine thereto, the membrane was reacted at 42 °C for 3 hours. After the membrane was washed 3 times with PBS - 0.1 % Tween 20 for 10 minutes, it was rinsed with 0.1 M Tris HCl (pH 9.5)-10 mM-MgCl₂ and then developed with BCIP - NBT system at room temperature for 20 minutes. The membrane was stored after washing with PBS - 0.1 % Tween 20.

### Results

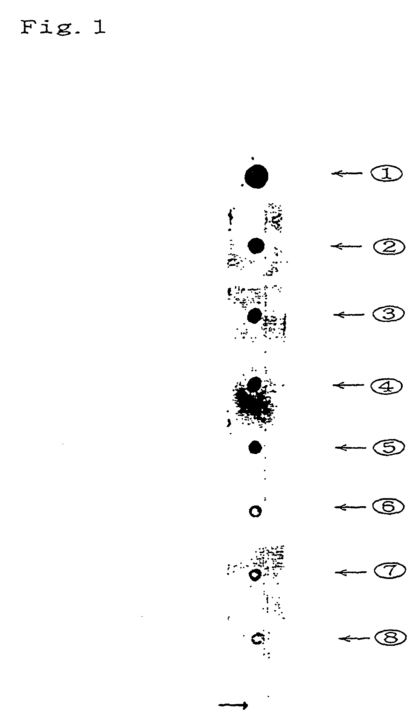

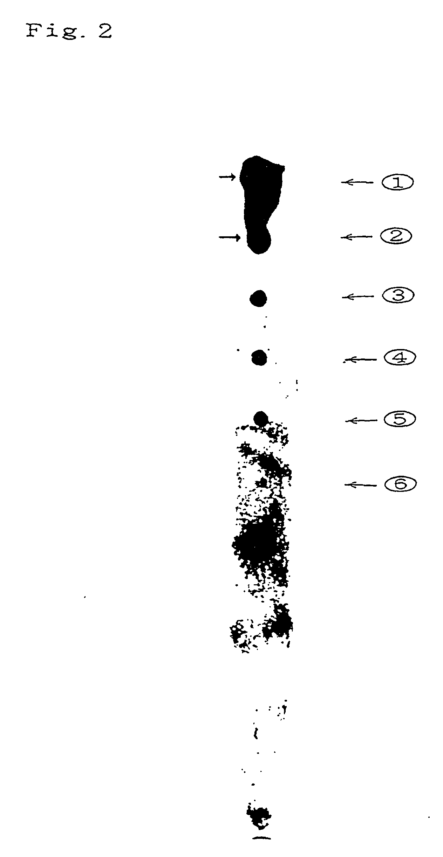

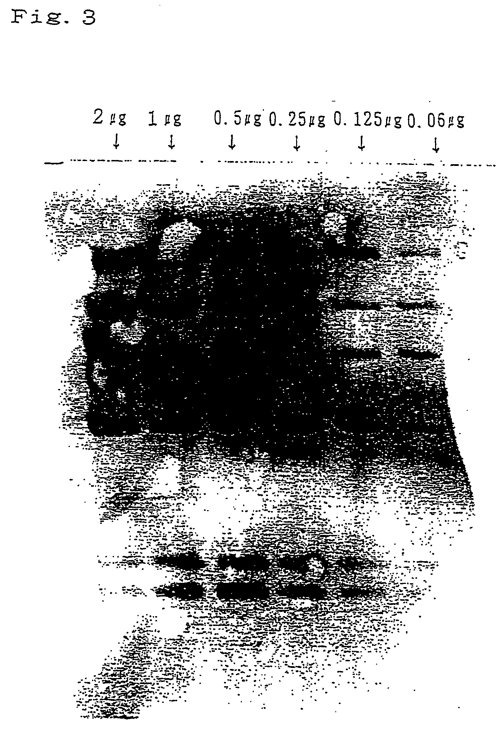

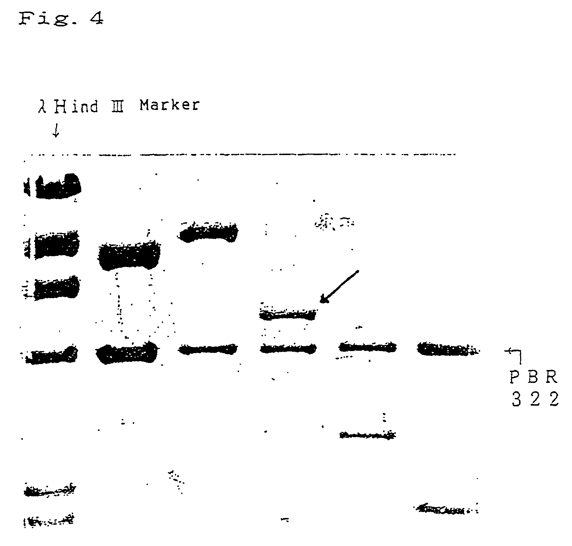

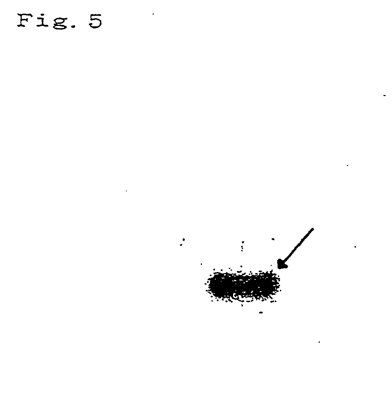

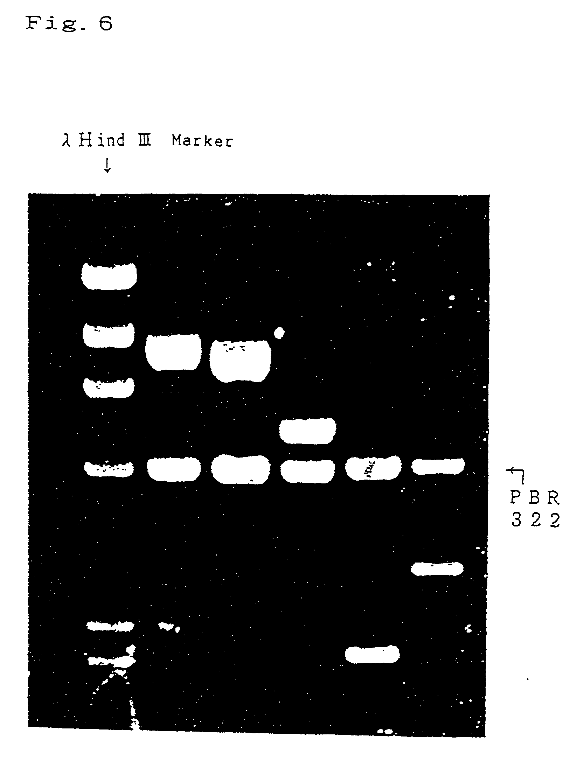

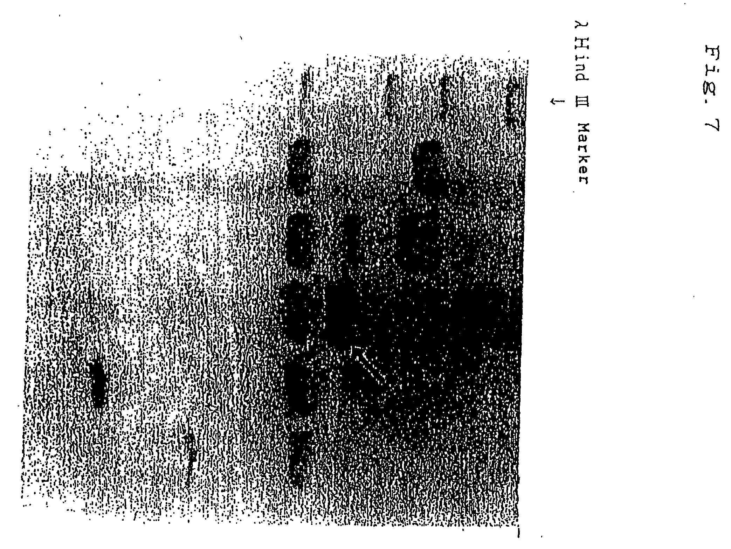

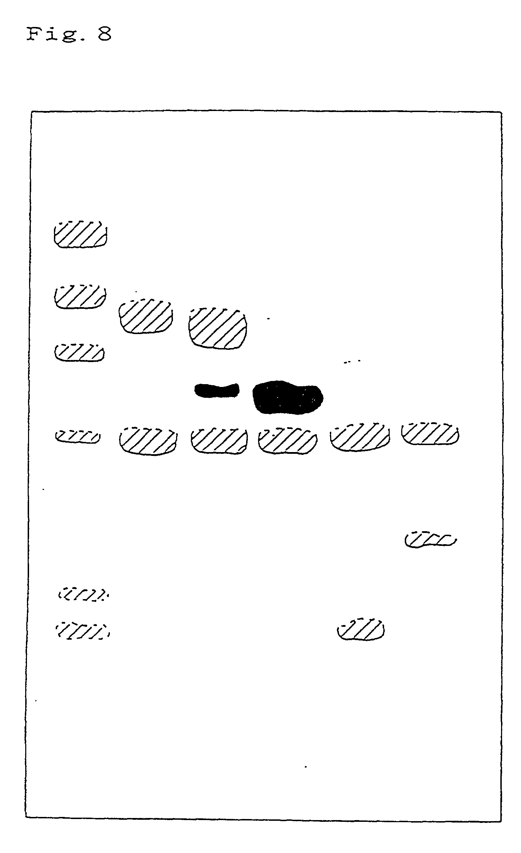

(i) λDNA or E. coli rRNA was dot-spotted and detected by alkaline phosphatase-labeled polyethyleneimine. The results are shown in Figures 1 and 2. The amounts of λDNA spots are
   (1) 4.7 µg, (2) 470 ng, (3) 47 ng, (4) 4.7 ng, (5) 470 pg, (6) 47 pg, (7) 4.7 pg, (8) 470 fg from the top in Fig. 1. The amounts of γRNA spots are (1) 5 µg, (2) 500 ng, (3) 50 ng, (4) 5 ng, (5) 500 pg, (6) 50 pg from the top in Fig. 2.
   (ii) After serial dilutions of λHind III DNA the DNA was fractionated by agarose gel electrophoresis, electro-blotted from gel to a membrane, each specimen was detected by alkalinephosphataselabeled polyethyleneimine. The results are shown in Fig. 3. The results showed that the profile obtained by ethydium bromide after agarose gel electrophoresis is completely identified with the pattern obtained by electroblotting of the specimen of the serial dilution (0.06 µg), and thus proved that the method of the invention is correct.
   (iii) DNA fragments inserted in pBR322, containing a size marker of λHind III DNA, were split with a restriction enzyme, fractionated by agarose gel electrophoresis, transferred on membrane by electoblotting, and hybridized with ³²P-DNA probe. The membrane was exposed to an X-ray film to obtain signals (Fig. 5). In order to identify a kind of hybridized DNA, the membrane was treated with peroxidase-labeled polyethyleneimine and positions of all blotted DNA were visualized (Fig.4).
   (iv) The DNA fragments which were treated as described in step
   (iii) were hybridized with Bio-DNA probe system to simultaneously detect both of hybridized DNA and non-hybridized DNA. By suitable choice of Bio-DNA probe and each enzyme in alkaline phosphatase-labeled streptavidin and peroxidase-labeled polyethyleneimine, a hybridized DNA and a non-hybridized DNA were differentiated by a double staining system. The results are shown in Fig. 7 and also, the difference in color is shown in Fig. 8. In the figure, the shaded portion represents brown color (the peroxidase-labeled polyethyleneimine system) and the black portion represents purple color. The patterns were identified with the results (Fig. 6) obtained by the ethydium bromide staining after the same treatment as described above.

## Claims

1. A method of detecting a nucleic acid which comprises bringing a solid carrier suspected to carry or contain a nucleic acid into contact with a polyamine to which a label capable of generating a detectable signal or a precursor thereof is bound, to form an electrostatically bonded complex between said nucleic acid and said polyamine, said precursor converting into said label, if used, removing the polyamine which has not formed any electrostatically bonded complex before or after the conversion of said precursor and then detecting said label.

2. A method of distinguishing a target nucleic acid and any nucleic acid other than said target nucleic acid combining the following steps (i) and (ii) in voluntary order, which comprises bringing a solid carrier suspected to carry or contain a target nucleic acid,
(i) (the first step) into contact with a probe to which a label capable of generating a detectable signal or a precursor thereof is bound, being capable of hybridizing with said target nucleic acid under hybridization conditions, to form a hybrid, said precursor converting into said label, if used, removing said probe which has not formed any hybrid before or after the conversion of said precursor and then detecting said label, and
(ii) (the second step) into contact with a polyamine to which a label capable of generating a detectable signal or a precursor thereof is bound, to form an electrostatically bonded complex between said nucleic acid and said polyamine, said precursor converting into said label, if used, removing said polyamine which has not formed any electrostatically bonded complex before or after the conversion of said precursor and then detecting said label.

3. The method of distinguishing a nucleic acid according to claim 2, wherein the first step is carried out by Southern blot hybridization.

4. The method according to any one of claims 1 to 3, wherein said label capable of generating a detectable signal or a precursor thereof is an enzyme.

5. The method according to claim 4, wherein said enzyme is alkaline phosphatase or peroxidase.

6. The method according to claim 4 or 5, wherein said enzyme binds to said polyamine through a crosslinking agent.

7. The method according to claim 6, wherein said crosslinking agent is benzoquinone.

8. The method according to any one of claims 1 to 7, wherein said polyamine is a naturally occurring polyamine or a synthetic polyamine, having an aliphatic chain of 3 to 50 carbon atoms and primary amino groups at both ends of said chain which may be interrupted by an imino group.

9. The method according to claim 8, wherein said polyamine is a polyamine having an aliphatic chain of 6 to 15 carbon atoms.

10. The method according to claim 9, wherein said polyamine is a synthetic polyamine.

11. The method according to claim 10, wherein said polyamine is polyethyleneimine.

12. A kit for detecting a nucleic acid comprising
(i) a polyamine-labeled enzyme and
(ii) a chromogen which generates a label by enzymatic action.

13. The kit for detecting a nucleic acid according to claim 12, wherein said enzyme is alkaline phosphatase or peroxidase.

14. The kit for detecting a nucleic acid according to claim 12 or 13, wherein said enzyme binds to said polyamine through a cross-linking agent.

15. The kit for detecting a nucleic acid according to claim 14, wherein said cross-linking agent is benzoquinone.

16. The kit for detecting a nucleic acid according to any one of claim 12 to 15, wherein said polyamine is a naturally occurring polyamine or a synthetic polyamine, having an aliphatic chain of 3 to 50 carbon atoms and primary amino groups at both ends of said chain which may be interrupted by an imino group.

17. The kit for detecting a nucleic acid according to claim 16, wherein said polyamine is a polyamine having an aliphatic chain of 6 to 15 carbon atoms.

18. The kit for detecting a nucleic acid according to claim 17, wherein said polyamine is a synthetic polyamine.

19. The kit for detecting a nucleic acid according to claim 18, wherein said polyamine is polyethyleneimine.

## Patentansprüche

1. Verfahren zum Nachweis einer Nucleinsäure, umfassend das Inkontaktbringen eines festen Trägers, von dem angenommen wird, daß er eine Nucleinsäure trägt oder enthält, mit einem Polyamin, an das ein Marker gebunden ist, der ein nachweisbares Signal erzeugen kann, oder ein Vorläufer davon, so daß ein elektrostatisch verbundener Komplex zwischen der Nucleinsäure und dem Polyamin erzeugt wird, die Umwandlung des Vorläufers, falls einer verwendet wurde, in den Marker, Entfernung des Polyamins, das keinen elektrostatisch verbundenen Komplex gebildet hat, vor oder nach der Umwandlung des Vorläufers und dann der Nachweis des Markers.

2. Verfahren zur Unterscheidung einer Zielnucleinsäure und einer beliebigen Nucleinsäure, die nicht die Zielnucleinsäure ist, durch Kombination der folgenden Schritte (i) und (ii) in beliebiger Reihenfolge, umfassend das Inkontaktbringen eines festen Trägers, von dem angenommen wird, daß er eine Zielnucleinsäure trägt oder enthält,
(i) (erster Schritt) mit einer Sonde, an die ein Marker gebunden ist, der ein nachweisbares Signal erzeugen kann, oder ein Vorläufer davon, die mit der Zielnucleinsäure unter Hybridisierungsbedingungen hybridisieren kann, so daß ein Hybrid erzeugt wird, Umwandlung des Vorläufers, falls einer verwendet wurde, in den Marker, Entfernen der Sonde, die kein Hybrid gebildet hat, vor oder nach der Umwandlung des Vorläufers und dann der Nachweis des Markers, und
(ii) (zweiter Schritt) mit einem Polyamin, an das ein Marker gebunden ist, der ein nachweisbares Signal erzeugen kann, oder ein Vorläufer davon, so daß ein elektrostatisch verbundener Komplex zwischen der Nucleinsäure und dem Polyamin erzeugt wird, Umwandeln des Vorläufers, falls einer verwendet wurde, in den Marker, Entfernen des Polyamins, das keinen elektrostatisch verbundenen Komplex gebildet hat, vor oder nach der Umwandlung des Vorläufers und dann Nachweis des Markers.

3. Verfahren zur Unterscheidung einer Nucleinsäure nach Anspruch 2, wobei der erste Schritt durch Southern-Blot-Hybridisierung durchgeführt wird.

4. Verfahren nach einem der Ansprüche 1 bis 3, wobei der Marker, der ein nachweisbares Signal erzeugen kann, oder ein Vorläufer davon ein Enzym ist.

5. Verfahren nach Anspruch 4, wobei das Enzym alkalische Phosphatase oder Peroxidase ist.

6. Verfahren nach Anspruch 4 oder 5, wobei das Enzym über ein Vernetzungsmittel an das Polyamin bindet.

7. Verfahren nach Anspruch 6, wobei das Vernetzungsmittel Benzochinon ist.

8. Verfahren nach einem der Ansprüche 1 bis 7, wobei das Polyamin ein natürlich vorkommendes Polyamin oder ein synthetisches Polyamin ist, das eine aliphatische Kette mit 3 bis 50 Kohlenstoffatomen und primäre Aminogruppen an beiden Enden der Kette aufweist, die durch eine Iminogruppe unterbrochen sein kann.

9. Verfahren nach Anspruch 8, wobei das Polyamin ein Polyamin mit einer aliphatischen Kette von 6 bis 15 Kohlenstoffatomen ist.

10. Verfahren nach Anspruch 9, wobei das Polyamin ein synthetisches Polyamin ist.

11. Verfahren nach Anspruch 10, wobei das Polyamin Polyethylenimin ist.

12. Kit zum Nachweis einer Nucleinsäure, umfassend
(i) ein Polyamin-markiertes Enzym und
(ii) ein Chromogen, das einen Marker durch enzymatische Wirkung erzeugt.

13. Kit zum Nachweis einer Nucleinsäure nach Anspruch 12, wobei das Enzym alkalische Phosphatase oder Peroxidase ist.

14. Kit zum Nachweis einer Nucleinsäure nach Anspruch 12 oder 13, wobei das Enzym über ein Vernetzungsmittel an das Polyamin bindet.

15. Kit zum Nachweis einer Nucleinsäure nach Anspruch 14, wobei das Vernetzungsmittel Benzochinon ist.

16. Kit zum Nachweis einer Nucleinsäure nach einem der Ansprüche 12 bis 15, wobei das Polyamin ein natürlich vorkommendes Polyamin oder ein synthetisches Polyamin mit einer aliphatischen Kette von 3 bis 50 Kohlenstoffatomen und primären Aminogruppen an beiden Enden der Kette ist, die durch eine Iminogruppe unterbrochen sein kann.

17. Kit zum Nachweis einer Nucleinsäure nach Anspruch 16, wobei das Polyamin ein Polyamin mit einer aliphatischen Kette von 6 bis 15 Kohlenstoffatomen ist.

18. Kit zum Nachweis einer Nucleinsäure nach Anspruch 17, wobei das Polyamin ein synthetisches Polyamin ist.

19. Kit zum Nachweis einer Nucleinsäure nach Anspruch 18, wobei das Polyamin Polyethylenimin ist.

## Revendications

1. Procédé pour détecter un acide nucléique, qui comprend les étapes suivantes : amener un support solide susceptible de porter ou de contenir un acide nucléique au contact d'une polyamine à laquelle un marqueur capable de produire un signal détectable ou un de ses précurseurs est lié, pour former un complexe électrostatiquement lié entre ledit acide nucléique et ladite polyamine, ledit précurseur se convertissant en ledit marqueur, si on l'utilise, éliminer la polyamine qui n'a pas formé de complexe électrostatiquement lié avant ou après la conversion dudit précurseur, puis détecter ledit marqueur.

2. Procédé pour distinguer un acide nucléique cible et un acide nucléique quelconque autre que ledit acide nucléique cible combinant les étapes (i) et (ii) suivantes dans un ordre volontaire, qui comprend les étapes suivantes : amener un support solide susceptible de porter ou de contenir un acide nucléique cible,
(i) (la première étape) au contact d'une sonde à laquelle un marqueur capable de produire un signal détectable ou un de ses précurseurs est lié, sonde qui est capable de s'hybrider avec ledit acide nucléique cible dans des conditions d'hybridation, pour former un hybride, ledit précurseur se convertissant en ledit marqueur, si on 1'utilise, éliminer ladite sonde qui n'a pas formé d'hybride avant ou après la conversion dudit précurseur puis, détecter ledit marqueur, et
(ii) (la seconde étape) au contact d'une polyamine à laquelle un marqueur capable de produire un signal détectable ou un de ses précurseurs est lié, pour former un complexe électrostatiquement lié entre ledit acide nucléique et ladite polyamine, ledit précurseur se convertissant en ledit marqueur, si on l'utilise, éliminer ladite polyamine qui n'a pas formé de complexe électrostatiquement lié avant ou après la conversion dudit précurseur puis, détecter ledit marqueur.

3. Procédé pour distinguer un acide nucléique selon la revendication 2, dans lequel la première étape est effectuée par hybridation par transfert de Southern.

4. Procédé selon l'une quelconque des revendications 1 à 3, dans lequel ledit marqueur capable de produire un signal détectable ou un de ses précurseurs est une enzyme.

5. Procédé selon la revendication 4, dans lequel ladite enzyme est une phosphatase alcaline ou une peroxydase.

6. Procédé selon la revendication 4 ou 5, dans lequel ladite enzyme se lie à ladite polyamine par l'intermédiaire d'un agent réticulant.

7. Procédé selon la revendication 6, dans lequel ledit agent réticulant est une benzoquinone.

8. Procédé selon l'une quelconque des revendications 1 à 7, dans lequel ladite polyamine est une polyamine naturelle ou une polyamine synthétique, ayant une chaîne aliphatique de 3 à 50 atomes de carbone et des groupes amino primaires aux deux extrémités de ladite chaîne qui peut être interrompue par un groupe imino.

9. Procédé selon la revendication 8, dans lequel ladite polyamine est une polyamine ayant une chaîne aliphatique de 6 à 15 atomes de carbone.

10. Procédé selon la revendication 9, dans lequel ladite polyamine est une polyamine synthétique.

11. Procédé selon la revendication 10, dans lequel ladite polyamine est une polyéthylèneimine.

12. Trousse pour détecter un acide nucléique comprenant
(i) une enzyme marquée avec une polyamine et
(ii) un chromogène qui produit un marqueur par action enzymatique.

13. Trousse pour détecter un acide nucléique selon la revendication 12, dans laquelle ladite enzyme est une phosphatase alcaline ou une peroxydase.

14. Trousse pour détecter un acide nucléique selon la revendication 12 ou 13, dans laquelle ladite enzyme se lie à ladite polyamine par l'intermédiaire d'un agent réticulant.

15. Trousse pour détecter un acide nucléique selon la revendication 14, dans laquelle ledit agent réticulant est une benzoquinone.

16. Trousse pour détecter un acide nucléique selon l'une quelconque des revendications 12 à 15, dans laquelle ladite polyamine est une polyamine naturelle ou une polyamine synthétique, ayant une chaîne aliphatique de 3 à 50 atomes de carbone et des groupes amino primaires aux deux extrémités de ladite chaîne qui peut être interrompue par un groupe imino.

17. Trousse pour détecter un acide nucléique selon la revendication 16, dans laquelle ladite polyamine est une polyamine ayant une chaîne aliphatique de 6 à 15 atomes de carbone.

18. Trousse pour détecter un acide nucléique selon la revendication 17, dans laquelle ladite polyamine est une polyamine synthétique.

19. Trousse pour détecter un acide nucléique selon la revendication 18, dans laquelle ladite polyamine est une polyéthylèneimine.
